# EUROPEAN PATENT SPECIFICATION

(11) **EP 1 625 234 B1**
(45) Date of publication and mention of the grant of the patent: **19.08.2009**
(21) Application number: 04760942.5
(22) Date of filing: 07.05.2004
(51) Int. Cl.: C12Q 1/68, C12P 19/34

(54) **BIOSYNTHETIC LABELING AND SEPARATION OF RNA**
BIOSYNTHETISCHE MARKIERUNG UND TRENNUNG VON RNA
ETIQUETAGE BIOSYNTHETIQUE ET SEPARATION DE L'ARN

(30) Priority: 09.05.2003 US 469439 P
(43) Date of publication of application: 15.02.2006
(73) Proprietor: THE BOARD OF TRUSTEES OF THE LELAND STANFORD JUNIOR UNIVERSITY, Palo Alto, CA 94306-1106 (US)
(72) Inventor: CLEARY, Michael, D., Eugene, Oregon 97401 (US); BOOTHROYD, John, Charles, Stanford, California 94305 (US)
(74) Representative: Brasnett, Adrian Hugh
(86) International application number: PCT/US2004/014457
(87) International publication number: WO 2004/101825

(56) References cited:
- US-A- 5 776 746
- US-B1- 6 251 596
- US-B1- 6 355 420
- MELVIN W T ET AL: "INCORPORATION OF 6-THIOGUANOSINE AND 4-THIOURIDINE INTO RNA APPLICATION TO ISOLATION OF NEWLY SYNTHESISED RNA BY AFFINITY CHROMATOGRAPHY" EUROPEAN JOURNAL OF BIOCHEMISTRY, BERLIN, DE, vol. 92, no. 2, 1978, pages 373-379, XP000914888 ISSN: 0014-2956
- WOODFORD T A ET AL: "SELECTIVE ISOLATION OF NEWLY SYNTHESIZED MAMMALIAN MRNA AFTER IN VIVO LABELING WITH 4-THIOURIDINE OR 6-THIOGUANOSINE" ANALYTICAL BIOCHEMISTRY, ACADEMIC PRESS, SAN DIEGO, CA, US, vol. 171, no. 1, 1988, pages 166-172, XP000914891 ISSN: 0003-2697
- CRAMER C L ET AL: "RAPID SWITCHING OF PLANT GENE EXPRESSION INDUCED BY FUNGAL ELICITOR" SCIENCE (WASHINGTON D C), vol. 227, no. 4691, 1985, pages 1240-1242, XP007902629 ISSN: 0036-8075
- STETLER G L ET AL: "MOLECULAR CLONING OF HORMONE RESPONSIVE GENES FROM THE YEAST SACCHAROMYCES-CEREVISIAE" PROCEEDINGS OF THE NATIONAL ACADEMY OF SCIENCES OF THE UNITED STATES OF AMERICA, vol. 81, no. 4, 1984, pages 1144-1148, XP007902624 ISSN: 0027-8424
- YI XIAOMING ET AL: "Both transcriptional and posttranscriptional mechanisms regulate human telomerase template RNA levels" MOLECULAR AND CELLULAR BIOLOGY, vol. 19, no. 6, June 1999 (1999-06), pages 3989-3997, XP007902625 ISSN: 0270-7306
- DIAMANDIS E P ET AL: "THE BIOTIN STREPT AVIDIN SYSTEM PRINCIPLES AND APPLICATIONS IN BIOTECHNOLOGY" CLINICAL CHEMISTRY, vol. 37, no. 5, 1991, pages 625-636, XP002905855 ISSN: 0009-9147
- PFEFFERKORN E R ET AL: "SPECIFIC LABELING OF INTRA CELLULAR TOXOPLASMA-GONDII WITH URACIL" JOURNAL OF PROTOZOOLOGY, vol. 24, no. 3, 1977, pages 449-453, XP008081128 ISSN: 0022-3921
- ILTZSCH M H ET AL: "Structure-activity relationship of ligands of uracil phosphoribosyltransferase from Toxoplasma gondii." BIOCHEMICAL PHARMACOLOGY 17 AUG 1994, vol. 48, no. 4, 17 August 1994 (1994-08-17), pages 781-792, XP007902634 ISSN: 0006-2952
- CLEARY MICHAEL D ET AL: "Biosynthetic labeling of RNA with uracil phosphoribosyltransferase allows cell-specific microarray analysis of mRNA synthesis and decay" NATURE BIOTECHNOLOGY, vol. 23, no. 2, February 2005 (2005-02), pages 232-237, XP007902626 ISSN: 1087-0156
- MATA ET AL: "Post-transcriptional control of gene expression: a genome-wide perspective" TRENDS IN BIOCHEMICAL SCIENCES, ELSEVIER, HAYWARDS, GB, vol. 30, no. 9, September 2005 (2005-09), pages 506-514, XP005041761 ISSN: 0968-0004
- MICHAEL BITTNER: "a window on the dynamics of biological switches" NATURE BIOTECHNOLOG, vol. 23, no. 2, - 2005 pages 183-184,

## Description

### INTRODUCTION

The most basic question one can ask in expression profiling is which genes have altered levels of transcription. This basic question lies at the heart of understanding the way that cells change in response to developmental signals; during oncogenesis; in response to injury or stress; and in performing day to day functions. Determining complex changes in gene transcription can lead to answers to complex questions - including the mechanisms of cellular life and death.

Transcriptional profiling is widely practiced; often being performed by hybridization of differentially labeled mRNA or cDNA to an array of polynucleotides. Methods for microarray fabrication include spotting of DNA onto nylon membranes or glass slides by robots with pins or ink jet printers. The DNA spotted corresponds to fragments of genomic DNA, cDNAs, PCR products or chemically synthesized oligonucleotides. cDNA arrays are often used in RNA expression analysis. Oligonucleotides can also be synthesized *in situ* on the surface of the array by means of light-directed combinatorial synthesis (photolithography) or ink jet technologies, which allow microarrays of higher density.

Transcriptional profiling experiments usually measure differential expression by the ratio of abundance levels between two samples. Genes with transcript abundance ratios above a fixed cut-off are said to be differentially expressed. Often replicate samples are included to control for biological variation. Positive and negative controls are essential in experimental design, for example by detecting expression of housekeeping genes, the use of mismatch oligonucleotide sequences for negative controls, and the like. There may be two experimental conditions or many, the conditions may be independent or related to each other in some way (as in a time series), or there may be many different combinations of experimental variables.

The ability to screen cells for differences in transcript abundance for a large number of different genes has advanced the molecular understanding of disease, and can lead to diagnostic and therapeutic applications. However, there are serious drawbacks to present methods, which limit the quality of information that can be obtained. Heretofore, it has not been possible to biosynthetically label eukaryotic RNA *in vivo* in such a way that newly synthesized RNA can be easily separated from other RNAs in the cell. As a result, transcriptional profiling has suffered from the limitation of only measuring differences in transcript abundance, not synthesis or decay. Current methods are thus slow to detect changes that occur when genes are switched off, because of the presence of large amounts of residual mRNA that must first be degraded to levels below the margin of error. Further, one cannot distinguish between mRNA made in different cells within a given tissue or sample unless those cells can be physically separated from each other.

Labeling of RNA *in vivo* has traditionally been hindered by the fact that nucleotides will generally not cross cell membranes and therefore cannot be incorporated into nascent mRNA without extensive manipulation of the cell, *e.g.* permeabilization of the cell membrane or isolation of nuclei. The present invention provides methods for resolving these issues.

### Relevant literature

Methods of using high density oligonucleotide arrays are known in the art. For example, Milosavljevic et al. (1996) Genomics 37:77-86 describe DNA sequence recognition by hybridization to short oligomers. The use of arrays for identification of unknown mutations is proposed by Ginot (1997) Human Mutation 10:1-10.

Quantitative monitoring of gene expression patterns with a complementary DNA microarray is described in Schena et al. (1995) Science 270:467. DeRisi et al. (1997) Science 270:680-686 explore gene expression on a genomic scale. Wodicka et a/. (1997) Nat Biotech 15:1-15 perform genome wide expression monitoring in S. *cerevisiae.*

UPRT is absent from mammalian cells but is present in certain protozoa, yeast, bacteria and plants. Previous studies on the UPRT of the protozoan *Toxoplasma gondii* tested 100 different compounds as potential substrates, analyzing the ability of the purified enzyme to convert these compounds into nucleoside monophosphates. In addition to two known toxic substrates (emimycin and 5-fluorouracil) only one other compound, 2,4-dithiouracil, was converted into UMP. litzsch and Tankersley (1994) Biochem Pharmacol. 48(4):781-92 describe structure-activity relationships of ligands of uracil phosphoribosyltransferase from *Toxoplasma gondii.* Cleary et al. (2002) Eukaryot Cell. 1(3):329-40 describe *Toxoplasma gondii* asexual development, including identification of developmentally regulated genes and distinct patterns of gene expression.

Melvin et al., European J. Biochem. 92(2), 373-379 (1978) describes a method for the isolation of newly synthesised RNA *in vitro* by treatment of cells in culture with 6-thioguanosine or 4-thiouridine, followed by separation of thiol-containing RNA by affinity chromatography on mercurated cellulose columns.

Woodford et al., Analytical Biochem. 171(1) 166-172 (1988) describes the *in vivo* labelling of mRNA from hamster cells with the thionucleoside analogs 4-thiouridine and 6-thioguanosine followed by selective recovery using phenylmercury affinity chromatography.

Cramer et al., Science 227(4691), 1240-1242 (2985) describes in *vivo* labelling of mRNA in cultured bean cells using 4-thiouridine and separation thereof using organomercurial affinity chromatography.

Stetler et al., PNAS 81 (4) 1144-1148 (1984) utilised incorporation of 4-thiouridine into nascent RNAs and their purification in a method for identifying yeast genes whose transcription is differentially regulated.

Yi Xiaoming et al., Mol. & Cell Biol. 19(6) 3989-3997 (1999) uses the incorporation of 4-thiouridine and selective isolation of labelled RNA by phenylmercury affinity chromatography to analyse the half-life of human telomerase RNA.

Pfefferkorn et al., J. Protozoology 24(3) 449-453 (1977) describes the use of [³H] uracil in the specific labelling of the nucleic acids of intracellular *T.gondii.*

### SUMMARY OF THE INVENTION

Methods are provided for differential biosynthetic labelling of RNA. The label thus introduced is a uracil analog selected from 2-thiouracil, 4-thiouracil, 2,4-dithiouracil, 2-thio-4-deoxyuracil, 5-carbethoxy-2-thiouracil, 5-carboxy-2-thiouracil, 5-(*n*-propyl)-2-thiouracil, 6-methyl-2-thiouracil and 6-(*n*-propyl)-2-thiouracil, each of which contains a reactive thiol moiety that provides a tag for quantitative separation of the RNA away from unlabelled RNA, or for the addition of a second moiety that provides for a detectable label.

Using this technique, RNA so labeled can be efficiently and specifically isolated away from all other RNA and analyzed, *e.g.* by hybridization methods such as "Northern" blots and microarray analysis. The RNA thus labeled can be used to quantitate newly synthesized RNA independent of any pre-existing RNA, and can rapidly and sensitively detect changes that occur when genes are switched on or off. The methods also allow distinction between mRNA made in different cells within a given tissue or sample, *e.g.* cells that have different functions, are infected *vs*. uninfected, or are from different host origins, for example in animals that are chimeric for a transgene. The methods of the invention are also useful for purification of specifically labeled RNA. The reactive thiol moiety permits determination of interaction between RNA and proteins, nucleic acids, and other molecules, *e.g.* by cross-linking of the moiety to nearby atoms.

Labeling is performed by using endogenous genes or introduced genetic sequences encoding a phosphoribosyl transferase or nucleoside kinase, which can specifically catalyze the transfer of the uracil analog into the corresponding nucleotide within a cell of interest. The cell of interest is contacted with the uracil analog, which crosses cell membranes and enters the cell. Once converted into the corresponding nucleotide, the analog remains in the cell to be triphosphorylated and incorporated into newly synthesized RNAs, thereby providing for highly selective labeling.

The uracil analog includes a thiol moiety, thereby providing a reactive moiety not normally present in nucleic acids. Other such moieties might include sulfonyl, nitro, chloro, bromo, fluoro, sulfamino, aza, e*ct*. Preferably the analog is not toxic to the cell. Preferred analog are thiouracil and 2,4 dithiouracil. The thiol moiety can readily react with a variety of linkers known in the art, permitting introduction of groups useful in separation and detection, e.g. haptens or molecules having known high affinity ligands, *e.g.* biotin, digoxigenin, *etc.;* specific labels, e.g. fluorescein, Cy3, Cy5, etc.; direct linking to substrate surfaces, e.g. capillaries, magnetic beads, microspheres; and the like.

In one embodiment of the invention, the phosphoribosyl transferase or nucleoside kinase coding sequences are under the transcriptional control of a regulated promoter. Promoters of interest include inducible promoter systems; developmentally regulated promoters; tumor specific promoters; tissue specific promoters, and the like. Alternatively, the promoter is constitutively expressed, and specificity of labeling is regulated by introduction of the purine or pyrimidine analog. In another embodiment of the invention, the coding sequences are present in a viral genome, and are expressed only in those cells infected by the virus. In another embodiment of the invention, the coding sequences are activated by a site specific recombinase, which in itself may be under control of a tissue-specific or otherwise regulated promoter.

### BRIEF DESCRIPTION OF THE DRAWINGS

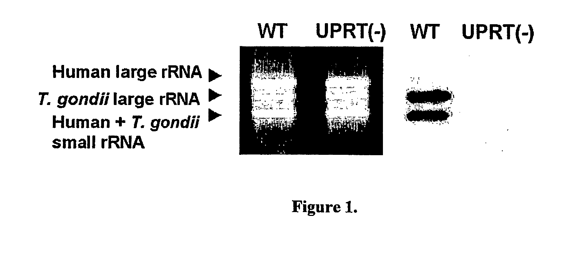

Figure 1. 2,4-dithiouracil specifically labels *Toxoplasma* RNA and labeling is dependent on UPRT. An ethidium bromide stained agarose gel is shown on the left and a northern blot of this gel, probed with streptavidin-HRP, is shown on the right. HPDP-Biotin labeled thiouracil containing RNA is only detected in the *Toxoplasma* ribosomal RNA and only in parasites with the UPRT enzyme. The faint signal detected above and below the major bands as a smear is *Toxoplasma* mRNA.

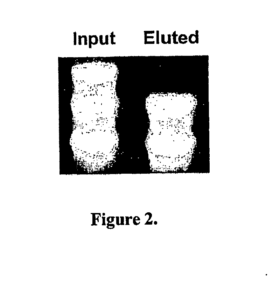

Figure 2. Streptavidin magnetic bead purification specifically isolates thiouracil containing RNA. 1.0 µg of input RNA (same sample but not purified on the streptavidin magnetic beads) was compared to 1.0 µg of the RNA eluted from this sample following purification. Only the *Toxoplasma* ribosomal bands are detectable in the eluted fraction.

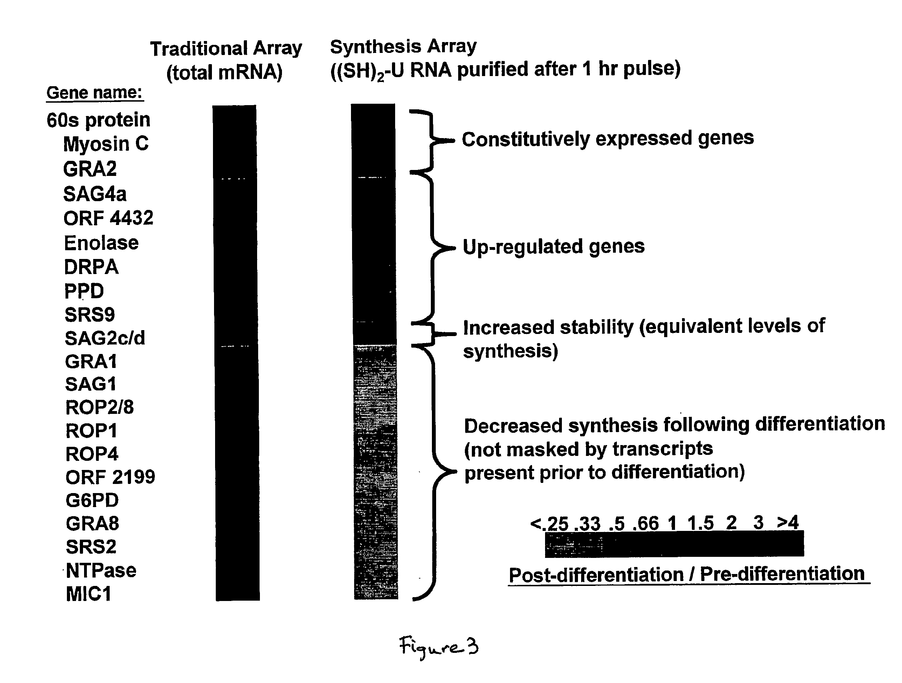

Figure 3. Synthesis arrays reveal developmental regulation not detectable by mRNA abundance measurement. These clusters show the relative mRNA levels in pre-differentiated or differentiated cells as determined by either traditional methods (mRNA abundance, left cluster) or the technique we have developed (mRNA synthesis, right cluster). The mRNA levels for a subset of genes from the microarray are shown as a ratio according to the scale shown at the bottom right of the figure. Red represents genes that are "induced" following differentiation and green represents genes that are "repressed" following differentiation.

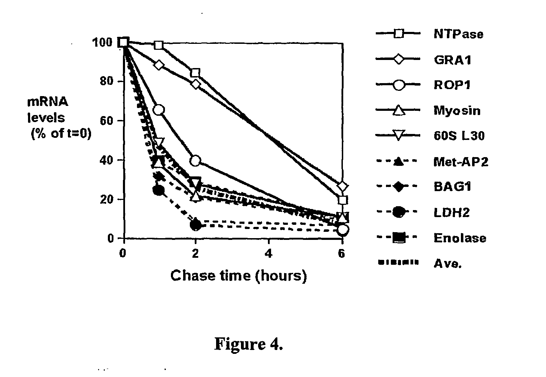

Figure 4. Relative mRNA decay rates determined by pulse-chase analysis. Following a one hour pulse with 2,4-dithiouracil, cells were grown in uracil and RNA was collected after 1, 2, and 6 hours of this uracil "chase". Thiouracil containing mRNA from each timepoint was hybridized to microarrays and the relative decay was determined for each gene. The graph shows the decay curves for a set of genes relative to the average decay for all genes on the microarrays.

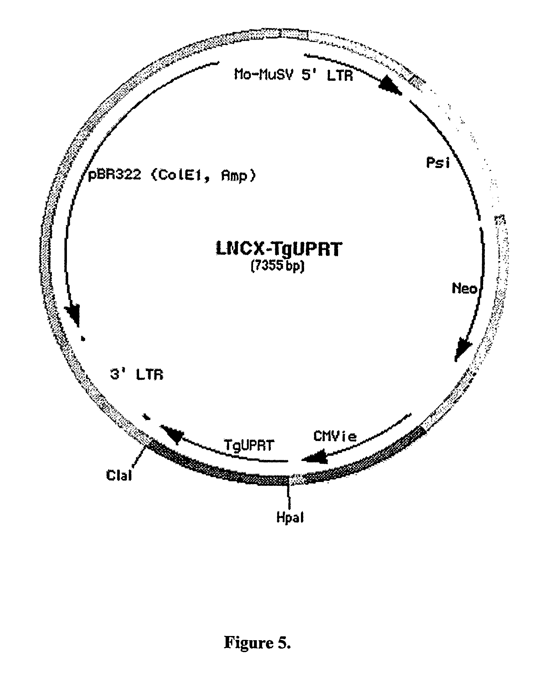

Figure 5. LNCX-TgUPRT construct for expression of *Toxoplasma* UPRT in human cells. The complete coding sequence of the *Toxoplasma* UPRT gene was PCR amplified from parasite cDNA and cloned into the LNCX retroviral vector using primers that introduced a Hpal restriction site at the 5' end of the gene and a Clal restriction site at the 3' end of the gene

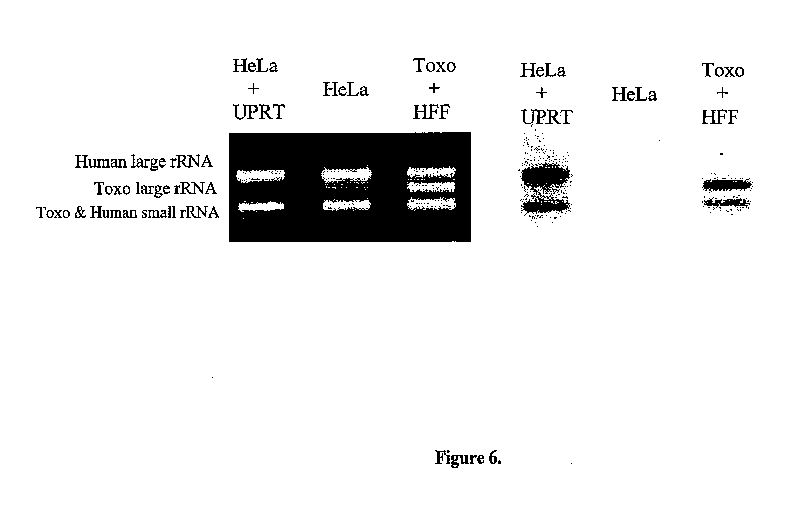

Figure 6. Expression of *Toxoplasma* UPRT in human cells permits incorporation of 2,4-dithiouracil into human RNA. An ethidium bromide stained agarose gel is shown on the left and the northern blot of this gel, probed with streptavidin-HRP, is shown on the right. Approximately equal amounts (0.5 ug) of RNA are loaded in all three lanes. HeLa cells expressing the Toxoplasma UPRT gene ("HeLa + UPRT") but not the unmodified HeLa cells ("HeLa") incorporate 2,4-dithioracil. As a control, *Toxoplasma-*infected human foreskin fibroblasts are shown. The major bands in these lanes represent the large and small subunit ribosomal RNAs. The smear in the "HeLa + UPRT" lane most likely represents HeLa mRNA, as demonstrated for Toxoplasma mRNA in results described above.

### DESCRIPTION OF THE SPECIFIC EMBODIMENTS

Methods are provided for biosynthetic labeling of RNA. The label specifically marks newly synthesized RNA in a cell of interest. RNA so labeled can be efficiently and specifically isolated away from all other RNA and analyzed, e.g. by hybridization methods such as "Northern" blots and microarray analysis. Labeling is performed by exploiting the natural presence of, or introducing gene sequences encoding, a phosphoribosyltransferase or nucleoside kinase that which can specifically incorporate uracil analogs into the corresponding nucleotide within a cell of interest. Although the nitrogenous bases can freely cross the cell membrane, once converted into the corresponding nucleotide, it remains in the cell to be incorporated into newly synthesized RNAs, providing for highly selective labeling.

The phosphoribosyltransferase or nucleotide kinase coding sequences are exogenous to the cell of interest, *i.e.* the sequence is not naturally present in the organism. Such sequences may be obtained from a different organism, or may be modified from naturally occurring sequences.

In one embodiment of the invention, the phosphoribosyltransferase or nucleotide kinase coding sequences are under the transcriptional control of a regulated promoter. Promoters of interest include inducible promoter systems; developmentally regulated promoters; tumor specific promoters; tissue specific promoters, and the like. Alternatively, the promoter is constitutively expressed, and specificity of labeling is regulated by introduction of the purine or pyrimidine analog. In another embodiment of the invention, the phosphoribosyltransferase or nucleotide kinase coding sequences are present in a viral genome, and are expressed only in those cells infected by the virus. In another embodiment of the invention, the phosphoribosyltransferase or nucleotide kinase coding sequences are activated by a site specific recombinase, which itself may be under control of a tissue-specific or otherwise regulated promoter.

*Enzymes.* Enzymes of interest for use in the methods of the invention include those enzymes in nucleotide salvage pathways. Salvage pathways take free bases, including adenine, guanine, hypoxanthine and uracil, and convert them to the corresponding nucleotides by phosphoribosylation. Enzymes active in salvage pathways include adenosine phosphoribosyltransferase (APRT), hypoxanthine-guanine phosphoribosyltransferase (HGPRT), and uracil phosphoribosyltransferase (UPRT). These enzymes have the EC classifications 2.4.2.5 (nucleoside ribosyltransferase); 2.4.2.7 (adenine phosphoribosyltransferase); 2.4.2.8 (hypoxanthine phosphoribosyltransferase); and 2.4.2.9 (uracil phosphoribosyltransferase). The salvage pathway for thymidine utilizes the enzyme thymidine kinase, including the enzyme classifications EC 2.7.1.21; EC 2.7.1.114; EC 2.7.1.118. Also included are, for example, adenosine kinase, cytidine kinase, uridine kinase, etc.

Preferably the substrate is non-toxic. Analogs comprising a thiol group are used in the methods of the invention because of advantages in terms of the chemistry of the resulting nucleic acid. The thiol moiety provides a unique reactive group for cross-linking, attachment of linkers, labeling, and the like. The uracil analog is provided as the free base.

An enzyme of particular interest is uracil phosphoribosyltransferase (UPRT). In a pyrimidine salvage pathway, uracil added to the medium is reacted with 5-phosphoribosyl-1-pyrophosphate to generate uridinemonophosphate. In addition to the naturally occurring substrates, enzymes of interest also catalyze the reaction with analogs of uracil having a reactive moiety not normally present in RNA, including 2,4 dithiouracil (2,4-dithiopyrimidine).
It may be noted that when commercially provided "2,4 dithiouracil" is provided to a cell, the subsequently synthesized mRNA may contain thiouracil, not dithiouracil, because the commercial material is in fact a mix of 2-thiouracil, 4-thiouracil and 2,4-dithiouracil and/or because of conversion of the dithiouracil to monothiouracil inside or outside the cells, before or after incorporation into the RNA. The term "thiouracil may be used herein to refer to the synthetic product. The enzyme may also utilize other uracil analogs, e.g. 5-fluorouracil; 6-azauracil; hydroxymethyl uracil; 6-(2-aminoethyl)amino-5-chlorouracil; 2-thiopyridine-*N*-oxide; *etc.*

The uracil phosphoribosyltransferase of *Toxoplasma gondii* is exemplary (see Donald and Roos (1995) P.N.A.S. 92:5749-5753; Genbank accession number 2114414A), although other enzymes may also find use. Enzymes with this activity have also been reported in a number of other prokaryotic and eukaryotic organisms, including prokaryotes, such as *Escherichia coli; Bacillus subtilis; Bacillus caldolyticus; Helicobacter pylori; Lactococcus lactis; Methanobacterium thermoautotrophicum; Mycoplasma pneumoniae; Mycobacterium bovis BCG; Streptococcus salivarius; Streptomyces tendae; Sulfolobus shibatae;* and protozoans, *e.g. Crithidia luciliae; Giardia intestinalis; Giardia lamblia; Plasmodium sp.; Tritrichomonas foetus; etc.* and yeast, *e.g. Candida albicans, Candida glabrata; Saccharomyces cerevisiae; etc.*

The suitability of a candidate enzyme for use in the methods of the invention may be empirically determined, using methods known in the art. Candidate enzymes can be selected based on similarity of amino acid sequence to a known phosphoribosyltransferases or thymidine kinases, by detection of biological activity, by selection from known enzymes, etc. The activity of the enzyme in transferring a purine or pyrimidine analog of interest into a nucleotide is readily determined using known assays, for example as described in the examples provided herein; as described by Iltzsch and Tankersley (1994) Biochem Pharmacol. 48(4):781-92; and the like.

The methods of the invention also include the use of a "variant" enzyme, which means a biologically active polypeptide as defined above, having less than 100% sequence identity with a naturally occurring enzyme. Such variants include polypeptides wherein one or more amino acid residues are added at the N- or C-terminus of, or within, the native sequence; from about one to forty amino acid residues are deleted, and optionally substituted by one or more amino acid residues; and derivatives of the above polypeptides, wherein an amino acid residue has been covalently modified so that the resulting product has a non-naturally occurring amino acid. Such variant polypeptides are functional, in that they retain the biological and/or biochemical activity of interest.

*Expression construct:* In the present methods, the enzyme of interest may be naturally present in an organism, or introduced in an expression construct. The DNA encoding the enzyme may be obtained from a cDNA library prepared from tissue expressing the mRNA; from a genomic library; by oligonucleotide synthesis; by PCR amplification using specific or consensus primers, and the like. As described above, there are many nucleotide salvage enzyme genetic sequences known in the art. Libraries may be screened with probes designed to identify the gene of interest or the protein encoded by it. Screening the cDNA or genomic library with the selected probe may be conducted using standard procedures as described in Sambrook et at, Molecular Cloning: A Laboratory Manual (New York: Cold Spring Harbor Laboratory Press, 1989). An alternative means to isolate the gene encoding UPRT is to use PCR methodology.

Amino acid sequence variants of enzymes are prepared by introducing appropriate nucleotide changes into the encoding DNA, or by synthesis of the desired protein. Such variants represent insertions, substitutions, and/or specified deletions of, residues within or at one or both of the ends of the amino acid sequence of a naturally occurring UPRT. Preferably, these variants represent insertions and/or substitutions within or at one or both ends of the mature sequence, and/or insertions, substitutions and/or specified deletions within or at one or both of the termini. Any combination of insertion, substitution, and/or specified deletion is made to arrive at the final construct, provided that the final construct possesses the desired biological activity.

The nucleic acid encoding the enzyme of interest is inserted into an integrating or replicable vector for expression. Many such vectors are available, including episomal vectors, integrating vectors, viral vectors, *etc*. The vector components generally include, but are not limited to, one or more of the following: an origin of replication, one or more marker genes, an enhancer element, a promoter, and a transcription termination sequence.

Expression vectors may contain a selection gene, also termed a selectable marker. This gene encodes a protein necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media.

Expression vectors contain a promoter that is recognized by the cell of interest, and is operably linked to the enzyme coding sequence. Promoters are untranslated sequences located upstream (5') to the start codon of a structural gene (generally within about 100 to 1000 bp) that control the transcription and translation of a particular nucleic acid sequence to which they are operably linked. Promoters may be inducible or constitutive, where inducible promoters broadly include promoters induced by a variety of developmental and environmental cues. Inducible promoters are promoters that initiate increased levels of transcription from DNA under their control in response to some change in conditions, *e.g*., the presence or absence of a nutrient, factor, developmental state, *etc*. A large number of promoters recognized by a variety of cells are well known.

Transcription from vectors in mammalian host cells may be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from mammalian promoters, *e.g.*, the actin promoter, PGK (phosphoglycerate kinase), or an immunoglobulin promoter, from heat-shock promoters, provided such promoters are compatible with the host cell systems. The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment.

The promoter used may be regulated by a pathway of interest, e.g. by the presence of a signaling molecule; tissue-specific; cell type-specific promoter; *etc.* For example, the promoter can be one designed to substantially specify expression within a specific tissue. Exemplary tissue-specific or cell-specific promoters include, but are not limited to, myosin heavy chain promoter for muscle specific expression, Madsen et al. (1998) Circ Res 82(8):908-917; lysosomal acid lipase promoter, Du et al. (1998) Gene 208(2):285-295; pancreatic expression using the amylase promoter, Dematteo et al. (1997) J Surg Res72(2):155-161; cardiac-specific overexpression, Kubota et al. (1997) Circ Res 81(4):627-635; folylpoly-gamma-glutamate synthetase promoter, Freemantle et al. (1997) J Biol Chem 272(40):25373-25379; tissue specific expression using neural restrictive silencer element, Kallunki et al. (1997) J Cell Biol 138(6):1343-1354 , placenta specific expression using the HGH promoter, Nogues et al. (1997) Endocrinology 138(8):3222-3227, expression during pregnancy using the prolactin promoter, Schuler et al. (1997) Endocrinology 138(8):3187-3194, tissue specific expression using the alpha1 (VI) collagen promoter, Braghetta et al. (1997) Eur J Biochem 247(1):200-208; B cell specific expression, Lennon et al. (1997) Immunogenetics 45(4):266-273; hypoxia induced expression, Gupta et al. (1996) Nucleic Acids Res 24(23):4768-4774; endothelium specific expression, Ronicke et al. (1996) Circ Res 79(2):277-285, the keratin promoters (e.g., human keratin 14 promoter (Wang et al. 1997 Proc Natl Acad Sci US 94:219-26); bovine cytokeratin gene promoters, BKIII and BKVI (Alexander et al. 1995 Hum Mol Genet 4:993-9); keratin 10 gene promoter (Bailleul et al. 1990 Cell 62:697-708); and tyrosinase promoters (specific for melanocytes)). Epidermal-specific promoters are reviewed in Fuchs et al. 1994 Princess Takamatsu Symp 24:290-302).

The expression can also be regulated by use of a site specific recombinase *e.g. cre* recombinase, FLP recombinase, pSR1 recombinase, *etc.* For example, a transcriptional inhibitor can be placed between two or more recombination sites. Induction of the recombinase will induce recombination between the sites, thereby deleting the inhibitor. The term "heterologous recombination site" is meant to encompass any introduced genetic sequence that facilitates site-specific recombination. In general, such sites facilitate recombination by interaction of a specific enzyme with two such sites. Exemplary heterologous recombination sites include, but are not necessarily limited to, lox sequences; recombination mediated by Cre enzyme; *frt* sequences (Golic et al. (1989) Cell 59:499-509; O'Gorman et al. (1991) Science 251:1351-5; recombination mediated by the FLP recombinase), the recognition sequences for the pSR1 recombinase of *Zygosaccharomyces rouxii* (Matsuzaki et a/. (1990) J. Bacteriol. 172:610-8), and the like. A lox site is a nucleotide sequence at which the gene product of the *ere* gene, catalyzes site-specific recombination. A particularly preferred lox site is a *loxP* site. The sequence of *loxP,* which is 34 bp in length, is known and can be produced synthetically or can be isolated from bacteriophage P1 by methods known in the art (see, *e.g*. Hoess et al. (1982) Proc. Natl. Acad. Sci. USA 79:3398). Other suitable lox sites include *loxB,* /ox*L*, and *loxR,* which can be isolated from E. *coli* (Hoess et al. (1982) Proc. Natl. Acad. Sci. USA 22:3398).

Transcription by higher eukaryotes is often increased by inserting an enhancer sequence into the vector. Enhancers are cis-acting elements of DNA, usually about from 10 to 300 bp, which act on a promoter to increase its transcription. Enhancers are relatively orientation and position independent, having been found 5' and 3' to the transcription unit, within an intron, as well as within the coding sequence itself. Many enhancer sequences are now known from mammalian genes (globin, elastase, albumin, α-fetoprotein, and insulin) and eukaryotic cell viruses, *e.g*. SV40 late enhancer, the cytomegalovirus early promoter enhancer, the polyoma late enhancer, adenovirus enhancers, *etc.* The enhancer may be spliced into the expression vector at a position 5' or 3' to the coding sequence, but is preferably located at a site 5' from the promoter.

Expression vectors for eukaryotic cells (yeast, protists, fungi, insect, plant, animal, human, or nucleated cells from other unicellular or multicellular organisms) will usually contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments that are transcribed and then post-transcriptionally processed by events such as splicing, polyadenylation, methylation and RNA editing.

Construction of suitable vectors containing one or more of the above-listed components employs standard ligation techniques. Isolated vectors or DNA fragments are cleaved, tailored, and re-ligated in the form desired to generate the vectors required. For analysis to confirm correct sequences in plasmids constructed, the ligation mixtures are used to transform host cells, and successful transformants selected by ampicillin or tetracycline resistance where appropriate. Vectors from the transformants are prepared, analyzed by restriction endonuclease digestion, and/or sequenced.

Episomal expression vectors may provide for the transient expression in mammalian cells. In general, transient expression involves the use of an expression vector that is able to replicate efficiently in a host cell, such that the host cell accumulates many copies of the expression vector and, in turn, synthesizes high levels of a desired polypeptide encoded by the expression vector.

Viral vectors of interest include, without limitation, retroviral vectors (*e.g*. derived from MoMLV, MSCV, SFFV, MPSV, SNV etc), lentiviral vectors (e.g. derived from HIV-1, HIV-2, SIV, BIV, FIV etc.), adeno- associated virus (AAV) vectors, adenoviral vectors (e.g. derived from Ad5 virus), SV40-based vectors, Herpes Simplex Virus (HSV)-based vectors *etc.* A vector construct may coordinately express the enzyme of interest and a marker gene such that expression of the marker gene can be used as an indicator for the expression of the enzyme of interest, as well as for analysis of gene transfer efficiency. This can be achieved by linking the test and a marker gene with an internal ribosomal entry site (IRES) sequence and expressing both genes from a single bi-cistronic mRNA. IRES sequence could be from a virus (e.g. EMCV, FMDV etc) or a cellular gene (e.g. elF4G, BiP, Kv1.4 etc). The examples of marker genes include drug resistance genes (neo, dhfr, hprt, gpt, bleo, puro etc) enzymes (ß-galactosidase, alkaline phosphatase, *etc.)* fluorescent genes (*e.g.* GFP, RFP, BFP, YFP) or surface markers (*e.g.* CD24, NGFr, Lyt-2 etc). A preferred marker gene is biologically inactive and can be detected by standard immunological methods. Alternatively, an "epitope tag" could be added to the test gene for detection of protein expression. Examples of such "epitope tags" are c-myc and FLAG (Stratagene).

Cells of interest are transfected or transformed with the above-described expression vectors. The genetic construct may be introduced into tissues or host cells by any number of routes, including calcium phosphate transfection, viral infection, microinjection, or fusion of vesicles. Jet injection may also be used for intramuscular administration, as described by Furth et al. (1992), Anal Biochem 205:365-368. The DNA may be coated onto gold microparticles, and delivered intradermally by a particle bombardment device, or "gene gun" as described in the literature (see, for example, Tang et al. (1992), Nature 356:152-154), where gold microprojectiles are coated with the DNA, then bombarded into cells. After introduction into the cell, the coding sequences may integrate into the host DNA, or be maintained as a replicable vector.

The transformed or transfected cells are cultured in conventional nutrient media modified as appropriate for inducing promoters, selecting transformants, or amplifying the genes encoding the desired sequences. Mammalian host cells may be cultured in a variety of media. Commercially available media such as Ham's F10 (Sigma), Minimal Essential Medium ((MEM), Sigma), RPMI 1640 (Sigma), and Dulbecco's Modified Eagle's Medium ((DMEM), Sigma) are suitable for culturing the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics, trace elements, and glucose or an equivalent energy source. Any other necessary supplements may also be included at appropriate concentrations that would be known to those skilled in the art. The culture conditions, such as temperature, pH and the like, are those previously used with the host cell selected for expression, and will be apparent to the ordinarily skilled artisan.

*Cells of Interest.* The methods of the present invention can employ naturally occurring cells and cell populations, genetically engineered cell lines, cells derived from transgenic animals, primary cells, normal and transformed cell lines, transduced cells and cultured cells, etc. Suitable cells include bacterial, fungal, protistan, plant and animal cells; *e.g.* avian; insect; reptilian; amphibian; mammalian; e.g. human, simian, rodent, etc. In one embodiment of the invention, the cells are mammalian cells; and may include complex mixtures of mammalian cells, *i.e.* where two or more cell types having distinguishable phenotypes are present. Examples of complex cell populations include naturally occurring tissues, for example blood, liver, pancreas, neural tissue, bone marrow, skin, and the like.

In addition, cells that have been genetically altered, e.g. by transfection or transduction with recombinant genes or by antisense technology, to provide a gain or loss of genetic function, may be utilized with the invention. Methods for generating genetically modified cells are known in the art, see for example "Current Protocols in Molecular Biology", Ausubel et al., eds, John Wiley & Sons, New York, NY, 2000. The genetic alteration may be a knock-out, usually where homologous recombination results in a deletion that knocks out expression of a targeted gene; or a knock-in, where a genetic sequence not normally present in the cell is stably introduced.

The expression vector can be used to generate transgenic organisms where the nucleic acid construct is randomly integrated into the genome. Vectors for stable integration include plasmids, retroviruses and other viruses, YACs, and the like. The modified cells or animals are useful in the study of gene function and regulation. For example, the enzyme of interest can be operably linked to a developmentally regulated promoter, and biosynthetically labeled mRNA used to study the regulation of gene expression, and analyze the expression profile of specific cells. Alternatively, the enzyme of interest can be regulated by a tissue specific promoter, or a promoter regulated in response to stimuli, e.g. neuronal signaling; antigen stimulation; hormone activation; exposure to toxins; etc.

For embryonic stem (ES) cells, an ES cell line may be employed, or embryonic cells may be obtained freshly from a host, e.g. mouse, rat, guinea pig, etc. Such cells are grown on an appropriate fibroblast-feeder layer or grown in the presence of leukemia inhibiting factor (LIF). When ES or embryonic cells have been transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of homologous recombination or integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting offspring screened for the construct. By providing for a different phenotype of the blastocyst and the genetically modified cells, chimeric progeny can be readily detected. The chimeric animals are screened for the presence of the modified gene and males and females having the modification can be mated to produce homozygous progeny or used as heterozygotes. The transgenic organism may be plants, fungus, protest, animal, etc., particularly any non-human mammal, such as laboratory animals, domestic animals, *etc*. The transgenic organism may be used in functional studies, drug screening, *etc*.

*Biosynthetic labeling.* The cells, tissue, or animal of interest is contacted with a uracil analog, which is usually provided in the form of the nitrogenous base, i.e. not as the corresponding nucleoside. Where the enzyme is operably linked to a regulated (inducible) promoter, the analog may be present in the medium, feed, *etc*., prior to induction and biosynthetic labeling. Where the enzyme is operably linked to a constitutive promoter, the analog will be added at the time biosynthetic labeling is to commence.

The uracil analog will be present in culture medium at a concentration of at least about 0.1 µM, usually at least about 1 µM, more usually at least about 5 µM, and not more than about 10 mM, usually not more than about 5 mM, and more usually not more than about 2.5 mM. Where the uracil analog is being provided to an animal, *e.g*. in drinking water, food, *etc*., the concentration will be appropriately increased to allow for losses and reduced bioavailability.

The uracil analog is maintained in the culture medium, *etc.* for a period of time sufficient to label the RNA of interest, and will vary depending on the purpose of the investigation. In some analyses, the uracil analog will be provided as a pulse-chase, where the initial exposure to the analog is followed by exposure to a high concentration of uracil, thereby providing a defined period of time when the RNA is biosynthetically labeled.

RNA is obtained from the cells by conventional methods. It is not necessary to separate the cells of interest from adjacent cells, although crude separation (*e.g*. surgical excision of a tissue) can facilitate subsequent manipulations. Solid tissue can be homogenized or otherwise broken apart, although it is not necessary. The cells are lysed to produce a suspension of RNA. Methods of isolating total mRNA are well known to those of skill in the art. For example, methods of isolation and purification of nucleic acids are described in detail in Chapter 3 of Laboratory Techniques in Biochemistry and Molecular Biology: Hybridization With Nucleic Acid Probes, Part I. Theory and Nucleic Acid Preparation, P. Tijssen, ed. Elsevier, N.Y. (1993) and Chapter 3 of Laboratory Techniques in Biochemistry and Molecular Biology: Hybridization With Nucleic Acid Probes, Part I. Theory and Nucleic Acid Preparation, P. Tijssen, ed. Elsevier, N.Y. (1993)).
For example, the total nucleic acid can be isolated from a = sample using an acid guanidinium-phenol-chloroform extraction method. For many purposes, mRNA is of interest, and may be separated with, for example, oligo-dT columns if it is obtained from cells that polyadenylate mRNA (see Sambrook et al., Molecular Cloning: A Laboratory Manual (2nd ed.), Vols. 1-3, Cold Spring Harbor Laboratory, (1989), or Current Protocols in Molecular Biology, F. Ausubel et al., ed. Greene Publishing and Wiley-Interscience, New York (1987)).

The resulting RNA preparation will include RNA comprising the uracil analog, and will usually include unlabeled RNA. The labeled RNA can be separated from unlabeled RNA, or can be differentially tagged with a detectable label, e.g. a fluorescent label, *etc*., for further use.

Conveniently, a reactive thiol moiety on the uracil analog is reacted to form a covalent bond to a tag group, where the tag group is a hapten or small molecule binding partner, *e.g.* digoxin, digoxigenin, FITC, dinitrophenyl, nitrophenyl, biotin, etc, or detectable label.

Detectable labels suitable for use in the present invention include any composition detectable by spectroscopic, photochemical, biochemical, immunochemical, electrical, optical or chemical means. Useful labels in the present invention include biotin for staining with labeled streptavidin conjugate, magnetic beads, fluorescent dyes, radiolabels (e.g., ³H, ¹²⁵I, ³⁵S, ¹⁴C, or ³²P), enzymes (*e.g.* horseradish peroxidase, alkaline phosphatase and others commonly used in an ELISA), and colorimetric labels such as colloidal gold (*e.g.* gold particles in the 40-80 nm diameter size range) or colored glass or plastic (*e.g.* polystyrene, polypropylene, latex, *etc*.) beads.

A wide variety of fluorescers can be employed either alone or, alternatively, in conjunction with quencher molecules. Fluorescers of interest fall into a variety of categories having certain primary functionalities. These primary functionalities include 1- and 2-aminonaphthalene, p,p'-diaminostilbenes, pyrenes, quaternary phenanthridine salts, 9-aminoacridines, p,p'-diaminobenzophenone imines, anthracenes oxacarbocyanine, marocyanine, 3-aminoequilenin, perylene, bisbenzoxazole, bis-p-oxazolyl benzene, 1,2-benzophenazin, retinol, bis-3-aminopyridinium salts, hellebrigenin, tetracycline, sterophenol, benzimidzaolylphenylamine, 2-oxo-3 -chromen, indole, xanthen, 7-hydroxycoumarin, phenoxazine, salicylate, strophanthidin, porphyrins, triarylmethanes and flavin. Individual fluorescent compounds which have functionalities for linking or which can be modified to incorporate such functionalities include, *e.g.,* dansyl chloride; fluoresceins such as 3,6-dihydroxy-9-phenylxanthhydrol; rhodamineisothiocyanate; N-phenyl 1-amino-8-sulfonatonaphthalene; N-phenyl 2-amino-6-sulfonatonaphthalene: 4-acetamido-4-isothiocyanato-stilbene-2,2'-disulfonic acid; pyrene-3-sulfonic acid; 2-toluidinonaphthalene-6-sulfonate; N-phenyl, N-methyl 2-aminoaphthalene-6-sulfonate; ethidium bromide; stebrine; auromine-0,2-(9'-anthroyl)palmitate; dansyl phosphatidylethanolamine; N,N'-dioctadecyl oxacarbocyanine; N,N'-dihexyl oxacarbocyanine; merocyanine, 4(3'pyrenyl)butyrate; d-3-aminodesoxy-equilenin; 12-(9'anthroyl)stearate; 2-methylanthracene; 9-vinylanthracene; 2,2'(vinylene-p-phenylene)bisbenzoxazole; p-bis[2-(4-methyl-5-phenyl-oxazolyl)]benzene; 6-dimethylamino-1,2-benzophenazin; retinol; bis(3'-aminopyridinium) 1,10-decandiyl diiodide; sulfonaphthylhydrazone of hellibrienin; chlorotetracycline; N(7-dimethylamino-4-methyl-2-oxo-3-chromenyl)maleimide; N-[p-(2-benzimidazolyl)-phenyl]maleimide; N-(4-fluoranthyl)maleimide; bis(homovanillic acid); resazarin; 4-chloro-7-nitro-2,1,3benzooxadiazole; merocyanine 540; resorufin; rose bengal; and 2,4-diphenyl-3(2H)-furanone. Specific fluorochromes of interest include fluorescein isothiocyanate (FITC), rhodamine, Texas Red, phycoerythrin, allophycocyanin, 6-carboxyfluorescein (6-FAM), 2',7' -dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N' ,N' -tetramethyl-6-carboxyrhodamine (TAMRA). Cyanine dyes are of particular interest as a detectable label. Cyanine dyes are synthetic dyes in which a nitrogen and part of a conjugated chain form part of a heterocyclic system, such as imidazole, pyridine, pyrrole, quinoline and thiazoles; including Cy3 and Cy5, which are widely used as labels. Such directly labeled RNA can be used in hybridization analysis without further manipulation.

The use of biotin is of particular interest. Biotin is a vitamin widely used in biotechnology for its ability to bind with extremely high affinity to avidin, streptavidin, neutravidin, captavidin; *etc.,* herein generically referred to as avidins. Avidins usually each bind four biotins per molecule with high affinity and selectivity, although monomeric derivatives may also find use. Dissociation of biotin from streptavidin is reported to be about 30 times faster than dissociation of biotin from avidin. Their multiple binding sites permit a number of techniques in which unlabeled avidin, streptavidin or NeutrAvidin biotin-binding protein can be used to bridge two biotinylated reagents. Biotin can be conjugated through various chemistries to molecules of interest.

Examples of biotin reagents that will react to form covalent bonds to a thiol moiety include commercially available reagents; e.g. maleimido-biotin; maleimido-lc-biotin; n-biotinyl-n-(3-maleimidopropionyl)-I-lysine; maleimido-peo3-biotin; HPDP-biotin (n-(6-(biotininamido)hexyl)-3'-(2'-pyridylthio)propionate); iodoacetyl-biotin (n-iodoacetyl-n-biotinylhexylenediamine); and the like. HPDP is of special interest as the disulfide linkage that it forms with the sulfhydryl is readily broken by reduction with agents such as dithiothreitol, 2-mercaptoethanol, etc. and so the original material can be restored to its original form, free of the tag, after purification.

Biotinylated RNA can be separated by affinity chromatography with a biotin binding partner, *e.g.* avidin, streptavidin, neutravidin; etc.; or can combined with a labeled biotin binding partner, *e.g.* Cy5-avidin; Cy3-avidin; and for purposes of, for example, *in situ* hybridization, can be combined with a radiolabeled or heavy metal labeled binding partner.

Biotin binding conjugates are extensively used as secondary detection reagents in microarrays, blot analysis, and the like. The biotinylated RNA is bound to a blot, array, cell section, *etc.* Detection is mediated by reagents including fluorochrome labeled avidins, enzyme-conjugated avidins plus a fluorogenic, chromogenic, or chemiluminescent substrate. Fluorescent avidin and streptavidin are extensively used in DNA hybridization techniques. Avidins can also be used as labels when conjugated to fluorescent polystyrene microspheres. Nanogold and colloidal gold conjugates find use as a label in light microscopy, and electron microscopy applications.

The use of enzyme-amplified immunodetection is a well-established standard technique. Most frequently, the enzymes of choice are horseradish peroxidase, alkaline phosphatase and *Escherichia coli* β-galactosidase because of their high turnover rate, stability, ease of conjugation and relatively low cost. Diaminobenzidine (DAB) can be used as a substrate with HRP, which generates a brown-colored polymeric oxidation product localized at HRP-labeled sites. The DAB reaction product can be visualized directly by bright-field light microscopy or, following osmication, by electron microscopy. Alternative substrates include fluorogenic, chromogenic and chemiluminescent substrates.

Where separation of the biosynthetically labeled RNA is of interest, affinity chromatography may be used. Affinity chromatography makes use of the highly specific binding sites usually present in biological macromolecules, separating molecules on their ability to bind a particular ligand. Covalent bonds attach the ligand to an insoluble, porous support medium in a manner that overtly presents the ligand to the protein sample, thereby using natural biospecific binding of one molecular species to separate and purify a second species from a mixture. Antibodies are commonly used in affinity chromatography.

Preferably a microsphere or matrix is used as the support for affinity chromatography. Such supports are known in the art and commercially available, and include activated supports that can be coupled to the linker molecules. For example, Affi-Gel supports, based on agarose or polyacrylamide are low pressure gels suitable for most laboratory-scale purifications with a peristaltic pump or gravity flow elution. Affi-Prep supports, based on a pressure-stable macroporous polymer, are suitable for preparative and process scale applications.

The binding partner for affinity chromatography can be any high affinity, usually noncovalent, interactor. Common binding partners are avidins, antibodies, and the like. The RNA sample is applied to the binding partner at a salt concentration that provides for specific binding, and is eluted off in a differential salt concentration, in the presence of free biotin or hapten, by reduction with dithiothreitol or other reducing agents; *etc.*

The separated RNA can be amplified prior to hybridization. If a quantitative result is desired, care must be taken to use a method that maintains or controls for the relative frequencies of the amplified nucleic acids. Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. Detailed protocols for quantitative PCR are provided in PCR Protocols, A Guide to Methods and Applications, Innis et al., Academic Press, Inc. N.Y., (1990).

Other suitable amplification methods include, but are not limited to polymerase chain reaction (PCR) (Innis, et al., PCR Protocols. A guide to Methods and Application. Academic Press, Inc. San Diego, (1990)), ligase chain reaction (LCR) (see Wu and Wallace, Genomics, 4: 560 (1989), Landegren, et al., Science, 241: 1077 (1988) and Barringer, et al., Gene, 89: 117 (1990), transcription amplification (Kwoh, et al., Proc. Natl. Acad. Sci. USA, 86: 1173 (1989)), and self-sustained sequence replication (Guatelli, et al., Proc. Nat. Acad. Sci. USA, 87: 1874 (1990)).

Another method of interest utilizes reverse transcriptase and a primer and a sequence encoding the phage T7 promoter to provide single stranded DNA template. A second DNA strand is polymerized using a DNA polymerase. After synthesis of double-stranded cDNA, T7 RNA polymerase is added and RNA is transcribed from the cDNA template. Successive rounds of transcription from each single cDNA template results in amplified RNA. This particular method is described in detail by Van Gelder et a/. (1990) Proc. Natl. Acad. Sci. USA, 87:1663-1667. It will be appreciated by one of skill in the art that the direct transcription method provides an antisense (aRNA) pool.

The separated RNA may be labeled with a detectable label. The label may be incorporated by any of a number of means well known to those of skill in the art, *e.g.* during an amplification step, or reverse transcription step. For example, polymerase chain reaction (PCR) with labeled primers or labeled nucleotides will provide a labeled amplification product. Alternatively, a label may be added directly to the original RNA sample, or to the amplification product after the amplification is completed. Means of attaching labels to nucleic acids include, for example nick translation or end-labeling by kinasing of the nucleic acid and subsequent attachment of a nucleic acid linker joining the sample nucleic acid to a label. Suitable labels include any of those listed above.

The label may be added to the sample nucleic acid prior to, or after the hybridization. Indirect labels are joined to the hybrid duplex after hybridization. Often, the indirect label is attached to a binding moiety that has been attached to the target nucleic acid prior to the hybridization. For example, the RNA may be biotinylated before the hybridization, and after hybridization, an avidin-conjugated fluorophore will bind the biotin bearing hybrid duplexes providing a label that is easily detected. For a detailed review of methods of labeling nucleic acids and detecting labeled hybridized nucleic acids see Laboratory Techniques in Biochemistry and Molecular Biology, Vol. 24: Hybridization With Nucleic Acid Probes, P. Tijssen, ed. Elsevier, N.Y., (1993)).

In some applications it is useful to directly label nucleic acid samples by end-labeling without having to go through an amplification, transcription or other nucleic acid conversion step. End labeling can be performed using terminal transferase (TdT), or by ligating a labeled oligonucleotide or analog thereof to the end of a target nucleic acid or probe. Other end-labeling methods include the creation of a labeled or unlabeled "tail" for the nucleic acid using ligase or terminal transferase, for example. The tailed nucleic acid is then exposed to a labeled moiety that will preferentially associate with the tail. The tail and the moiety that preferentially associates with the tail can be a polymer such as a nucleic acid, peptide, or carbohydrate. The tail and its recognition moiety can be anything that permits recognition between the two, and includes molecules having ligand-substrate relationships such as haptens, epitopes, antibodies, enzymes and their substrates, and complementary nucleic acids and analogs thereof.

*Hybridization Analysis.* The labeled or separated RNA can be used in a variety of hybridization protocols, as known and widely practiced in the art. Procedures of particular interest include hybridization to arrays of polynucleotide probes. Hybridization of the labeled sequences is accomplished according to methods well known in the art. Hybridization can be carried out under conditions varying in stringency, preferably under conditions of high stringency, *e.g.* 6X SSPE, 65^{>}C, to allow for hybridization of complementary sequences having extensive homology.

High density microarrays of oligonucleotides are known in the art and are commercially available. The sequence of oligonucleotides on the array will correspond to the known target sequences of one of the genomes, as previously described. Arrays of interest may comprise at least about 10³ different sequences, at least about 10⁴ different sequences, and may comprise 10⁵ or more different sequences. The probes on the array may be oligonucleotides, *e.g.* from about 12 to 70 nucleotides in length, or may be larger sequences, e.g. cDNAs and fragments thereof. In a preferred embodiment, the microarrays used in the present methods are gene expression probe arrays. Such arrays comprise oligonucleotide probes derived from the sequence of open reading frames in the genome of interest. Commercially available high-density arrays containing a large number of oligonucleotide probes from genomic DNA sequence have been designed and used to monitor genome-wide gene expression, *e.g.* in mouse, human, *etc.*

Microarrays can be scanned to detect hybridization of the labeled samples. Methods and devices for detecting fluorescently marked targets on devices are known in the art. Generally such detection devices include a microscope and light source for directing light at a substrate. A photon counter detects fluorescence from the substrate, while an x-y translation stage varies the location of the substrate. A confocal detection device that may be used in the subject methods is described in U.S. Patent no. 5,631,734. A scanning laser microscope is described in U.S. patent no. 5,445,934, *supra.* A scan, using the appropriate excitation line, is performed for each fluorophore used. The digital images generated from the scan are then combined for subsequent analysis. For any particular array element, the ratio of the fluorescent signal from one nucleic acid sample is compared to the fluorescent signal from the other nucleic acid sample, and the relative signal intensity determined.

Specific uses of the methods of the invention include the determination of cellular sequences that are transcribed during viral infection. In this embodiment, the UPRT coding sequences is present in a virus. Upon infection of a cell of interest, the UPRT is expressed, *e.g.* during early stage, late stage, *etc.* Upon expression and contact with the uracil analog, all actively transcribed genes in the infected cell and the virus will be biosynthetically labeled. Labeled RNA derived from such cells will specifically define those genes expressed during virus infection, even when the majority of cells in the population are not infected.

RNA can be isolated following a pulse period in order to measure synthesis (independent of RNAs present before the pulse) of transcripts, *e.g.* those corresponding to all genes present on a microarray. Chasing with the counterpart purine or pyrimidine (lacking the reactive moiety) following the pulse will allow determination of the decay rates, *e.g.* via microarray analysis, through measurement of the decline in the abundance of the tagged RNA as a function of time following addition of the unlabelled chase reagent.

Selective expression of the phosphoribosyltransferase or thymidine kinase allows purification of RNA from only those cells able to incorporate the purine or pyrimidine analog, as in a mixture of cell types, a chimeric animal, or a virus engineered to express the enzyme of interest.

For convenience, kits may be supplied which provide the necessary reagents in a convenient form and together. For example kits could be provided that include a vector containing an enzyme of interest, e.g. UPRT, HGPRT, APRT, TK, which may be provided with a promoter, or with a cassette for insertion of a promoter of interest. Kits may further comprise reagents including a purine or pyrimidine analog useful with the enzyme, e.g. thiouracil with UPRT; thiopurine with HGPRT; etc.; biotin conjugated to an appropriate linker, e.g. HPDP-biotin; avidin labels or resins; and/or suitable buffers. Chips containing an appropriate microarray for the subject to be analyzed may also be included. Other components such as automated systems for determining and interpreting the hybridization results, software for analyzing the data, or other aids may also be included depending upon the particular protocol which is to be employed.

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention which will be limited only by the appended claims.

As used herein the singular forms "a", "an", and "the" include plural referents unless the context clearly dictates otherwise. Thus, for example, reference to "a cell" includes a plurality of such cells and reference to "the array" includes reference to one or more arrays and equivalents thereof known to those skilled in the art, and so forth. All technical and scientific terms used herein have the same meaning as commonly understood to one of ordinary skill in the art to which this invention belongs unless clearly indicated otherwise.

All publications are mentioned herein for the purpose of describing and disclosing, for example, the enzymes, constructs, and methodologies that are described in the publications, which might be used in connection with the presently described invention. The publications discussed above and throughout the text are provided solely for their disclosure prior to the filing date of the present application. Nothing herein is to be construed as an admission that the inventors are not entitled to antedate such disclosure by virtue of prior invention.

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (*e.g.* amounts, temperature, concentrations, *etc.*) but some experimental errors and deviations should be allowed for. Unless otherwise indicated, parts are parts by weight, molecular weight is average molecular weight, temperature is in degrees centigrade; and pressure is at or near atmospheric.

### EXPERIMENTAL

### Example 1

### Incorporation of thiouracil into RNA

*Methods and Results:* Initial experiments assayed the growth of *T. gondii* within human foreskin fibroblast cells in media containing a range of concentrations of 2,4-dithiouracil (5 µM to 2 mM). Equivalent numbers of parasites were used to infect host cells and the replication of the parasites in the different concentrations of 2,4-dithiouracil was monitored over time. No growth defects were observed for either the parasites or human cells at even the highest concentration (2 mM) of 2,4-dithiouracil.

To test for the incorporation of thiouracil into RNA, wild type (WT) parasites and mutants that lack the UPRT enzyme (UPRT(-)) were used to infect human foreskin fibroblasts in culture after which, the normal medium was replaced with medium containing 2 mM 2,4-dithiouracil. Following growth in this medium for 6 hours, the infected cells were harvested and RNA prepared by standard methods using Trizol reagent from Gibco-BRL.

The resulting RNA was incubated with HPDP-biotin (EZ-Link Biotin-HPDP (N-(6-(Biotinamido)hexyl)-3'-(2'-pyridyldithio)-propionamide) from Pierce Biotechnology) which specifically reacts with free thiols which are normally not present in RNA. For both RNA samples (WT and UPRT(-)) 25 µg of RNA was incubated with 50 µl of a 1 mg/ml Biotin-HPDP solution and TrisHCl/EDTA buffer (pH 7.4) for 1.5 hours. Biotinylated RNA was precipitated from any excess biotin by isopropanol precipitation and centrifugation. Approximately 2.5 µg of this material was resolved by agarose gel electrophoresis and transferred to a nitrocellulose filter, all by standard procedures. The resulting filter was then probed with streptavidin conjugated to horseradish peroxidase (Streptavidin-HRP) and the bound material detected with chemiluminescence (using the ECL kit from Amersham). The results (Figure 1) show that two of the three major bands detectable by ethidium staining of the RNA have thiouracil incorporated into them. These bands have previously been shown to correspond to the two major ribosomal RNAs of Toxoplasma. The upper-most major band is from the large subunit of the human ribosome. The absence of signal in this band indicates that the 2,4-dithiouracil is not incorporated into the human RNA as predicted since these human cells are not known to possess the means to salvage uracil or its derivatives from the medium.

### Example 2

### Specific Purification of thiouracil-labeled RNA Using Streptavidin Beads.

The above results demonstrate that thiouracil containing RNA can be specifically biotinylated. To separate this RNA from non-biotinylated RNA (i.e. all the RNAs made prior to the pulse with (SH)2-uracil) we have used a method of purification in which biotinylated RNA is bound to streptavidin attached to magnetic beads. Because biotinylation of the thiouracil is via a disulfide bond, the biotinylated RNA can be eluted from the streptavidin-magnetic beads by breaking this bond with a reducing agent (dithiothreitol or DTT).

*Methods and Results:* Toxoplasma were grown in human foreskin fibroblasts for 40 hours in either regular media or media containing 2 mM 2,4-dithiouracil. RNA was extracted and reacted with HPDP-biotin as described above. 50 µg of biotinylated RNA from each sample was incubated with 250 µl of streptavidin-magnetic porous glass beads (MPG Streptavidin from CPG Biotech) in a buffer containing 1M NaCl, 10mM EDTA. Following a 15 minute incubation at room temperature, the beads and any bound RNA were collected in a magnetic stand and the supernatant removed. The beads were then washed three times in 300 µl of buffer containing 1M NaCl, 10mM EDTA, and 1% SDS followed by two washes in 1M NaCl, 10mM EDTA. Biotinylated RNA was eluted following two rounds of incubation in 250 µl of 100 mM DTT for 5 minutes at room temperature. Eluted RNA was precipitated following addition of 50 µl of 5M NaCl, 500 µl of isopropanol, and 2 µg glycogen. As expected, no detectable RNA (as measured by spectrophotemetry and ethidium staining of an agarose gel) was eluted from the RNA prepared from parasites grown in the absence of 2,4-dithiouracil. This result provides evidence that this purification is specific for biotinylated thiouracil containing RNA. Additionally, only Toxoplasma ribosomal RNA was eluted from the RNA prepared from parasites grown in human cells in the presence of 2,4-dithiouracil (Figure 2). This result further demonstrates that this purification is specific for biotinylated thiouracil containing RNA.

### Example 3.

### Thiouracil is Incorporated Into mRNA and this mRNA Can be Used in Microarray Experiments

Having shown that the Toxoplasma ribosomal RNA could be specifically labeled and purified, we next asked if the parasite's mRNA could also be purified and whether this mRNA could be used in microarray experiments. To do this, we used the thiouracil-labeled RNA that had been specifically eluted from the streptavidin beads as a substrate for reverse transcriptase (i.e., cDNA synthesis) in the presence of cy5-labeled nucleotides. This material was used in microarray experiments and compared to results from a separate microarray in which RNA from parasites grown in the absence of 2,4-dithiouracil was made into cDNA in the presence of cy5 labeled nucleotides.

*Methods and Results:* The two RNAs were compared for their ability to bind to spotted cDNA probes corresponding to Toxoplasma genes in a microarray as previously described. 2 µg of RNA from both samples was labeled with cy5 during first strand cDNA synthesis and each cy5 labeled sample was mixed with a common cy3 reference probe and hybridized to separate microarrays. The data (Figure 3) showed that the thiouracil-labeled mRNA served well as a template for the cDNA synthesis and that the hybridization was globally similar to that seen with unlabeled mRNA, as expected since the incorporation of thiouracil was over a 40 hour period and thus would be expected to completely saturate all mRNAs.

### Example 4.

### Measurement of mRNA Synthesis on Microarrays

An advantage of the methods of the invention is that by using short time-pulses of labeling, only mRNA synthesized during that period will be used for cDNA synthesis and thus mRNA that existed prior to the pulse-label period will not contribute to the signal. In cases where a particular condition causes a given gene to stop being transcribed, if labeling is started after that condition has been implemented, no mRNA for that gene will be labeled and a dramatic difference will be seen compared to mRNA labeled in the untreated control. With normal array technology (using cDNA synthesized off total mRNA present before and after the change in condition) the signal might show no significant decrease after the change because the mRNA made before the change persists for a considerable length of time. Thus this method gives a much more sensitive analysis of changes in gene expression because it measures mRNA synthesis rather than mRNA abundance.

In addition, since mRNA abundance is dependent on synthesis and decay, and since we can measure abundance and synthesis, decay can now be deduced. For example, if the abundance of a given mRNA drops substantially and its synthesis plummets to zero, the decay can be deduced to be slower than the decay for a gene whose abundance follows the same curve but whose synthesis is unchanged. Likewise, for a gene whose abundance increases, we can now determine whether this increase is due to an increase in synthesis and little or no change in decay or no change in synthesis and a decrease in decay.

*Methods and Results:* To compare results obtained by measurement of mRNA abundance versus measurement of mRNA synthesis, microarray analysis was performed using either total mRNA or purified thiouracil RNA following a one hour pulse. Total and pulse-labeled mRNAs were prepared from parasites grown in human foreskin fibroblast cultures maintained in either neutral pH media or high pH media. Growth in high pH induces parasites to develop into a developmental stage known as a bradyzoite while parasites grown in neutral pH grow as a distinct stage known as a tachyzoite. Following four hours of growth as tachyzoites, cultures of parasites were either kept in neutral pH media for 48 hrs or switched to high pH media for 72 hours. Comparison of gene expression between tachyzoites and bradyzoites at these timepoints has been published, using traditional microarray techniques (i.e. mRNA abundance measurements). At the end of each timepoint, 48 or 72 hours, the media was removed and media containing 2 mM 2,4-dithiouracil (at the corresponding pH) was added for one hour. At the end of this hour, RNA was prepared using Trizol and mRNA was purified using the FastTrak mRNA isolation kit from Invitrogen. An aliquot from each mRNA sample was used in microarray analysis of total mRNA abundance. The remaining mRNA was biotinylated and purified over streptavidin magnetic beads as described above. This RNA was used in microarray experiments to measure the mRNA synthesized during the one hour 2,4-dithiouracil pulse. In all microarray experiments, 150 ng of Toxoplasma mRNA was labeled with cy5 dUTP and compared to a common cy3 labeled reference sample. The ratio of mRNA in bradyzoites and tachyzoites was determined for both the total abundance and the pulse-labeled mRNA microarrays, using previously described analysis methods.

Comparison of the results obtained when measuring mRNA abundance to the results obtained when measuring synthesis reveal several differences between these methods of microarray analysis and demonstrate the power of measuring only mRNAs synthesized during a short pulse. Figure 4 shows that while some genes have apparently similar levels of induction in bradyzoites when measuring mRNA abundance, there are significant differences in changes in the mRNA synthesis rates (and by inference, the decay rates) for these genes. These results also demonstrate that a number of genes that appear to show little or no change in their respective mRNA abundance between the two stages actually have significantly decreased levels of synthesis in bradyzoites. This regulation is detectable in the synthesis arrays because transcripts synthesized before the 2,4-dithiouracil pulse were removed prior to the labeled cDNA synthesis. This result demonstrates a solution to what is otherwise a serious limitation of traditional microarrays; no change can be detected for these genes when measuring mRNA abundance because transcripts present before addition of the high pH media have not decayed enough to allow detection of any difference between the two stages of the parasite.

### Example 5.

### Transgenic Expression of Toxoplasma gondii UPRT Permits Use of this Technique in Cells that Normally Lack UPRT Activity.

To show that this approach can be used in organisms and/or cell lines that normally lack UPRT activity, we introduced the Toxoplasma UPRT gene into HeLa cells, a cell line of human origin. Expression of this gene in any cell type should be all that is required to perform the same experiments shown above in an organism other than *Toxoplasma gondii.*

*Methods and Results:* The coding region of the Toxoplasma UPRT gene was incorporated into the retroviral vector known as LNCX (Miller & Rosman (1989) BioTechniques 7:980-990), placing the UPRT gene under control of the cytomegalovirus immediate-early promoter. The resulting construct is shown in Figure 5. This construct was introduced into HeLa cells and cell lines that had stably incorporated the vector were selected using neomycin. Cells that survived this selection and non-transfected HeLa cells were both grown in the presence of 2,4-dithiouracil for 6 hours. RNA was isolated from these cells and labeled with HPDP-biotin as described above. Incorporation of thiouracil was detected by northern blot as described above. The results (Figure 6) show that the normal HeLa cells lack the ability to incorporate the thiouracil. The HeLa cells that had received the Toxoplasma UPRT gene, however, showed good incorporation at a level similar to that seen with the parasites on a per microgram of RNA basis.

## Claims

1. A method of biosynthetically labeling RNA in a cell of interest, the method comprising:
contacting said cell *in vitro* with a uracil analog not normally present in RNA and selected from 2-thiouracil, 4-thiouracil, 2,4-dithiouracil, 2-thio-4-deoxyuracil, 5-carbethoxy-2-thiouracil, 5-carboxy-2-thiouracil, 5-(*n*-propyl)-2-thiouracil, 6-methyl-2-thiouracil and 6-(n-propyl)-2-thiouracil, wherein said cell comprises a phosphoribosyltransferase or nucleoside kinase that can specifically incorporate said uracil analog into the corresponding nucleotide, wherein said phosphoribosyltransferase or nucleoside kinase is exogenous to the cell of interest, and wherein said uracil analog is incorporated into RNA synthesized by said cell; and obtaining RNA comprising said thiol moiety from said cell.

2. The method according to claim 1, wherein said uracil analog is selected from 2-thiouracil, 4-thiouracil, 2,4-dithiouracil and mixtures thereof.

3. The method according to claim 1, wherein said method further comprises conjugating a small molecule binding partner to the thiol component of the uracil analog.

4. The method according to Claim 3, wherein said small molecule binding partner is biotin and/or wherein said small molecule binding partner is conjugated to a detectable label which is, for example, a fluorochrome, radiolabel, heavy metal label, or enzyme conjugate.

5. The method according to Claim 4, further comprising the step of binding a specific binding partner to said small molecule binding partner, optionally wherein either:
said specific binding partner is conjugated to an insoluble substrate for affinity chromatography, and wherein said biosynthetically labelled RNA is separated from non-labelled RNA; or
said specificy binding partner is conjugated to a detectable label, for example a fluorochrome, radiolabel, heavy metal label or enzyme conjugate.

6. The method according to Claim 5, wherein said separated RNA is reverse transcribed or wherein said separated RNA is amplified; and/or wherein said separated RNA is labelled, optionally during amplification, with a detectable label, by, for example, end-labelling or by reverse transcriptase.

7. The method of claim 1 wherein said phosphoribosyltransferase is a uracil phosphoribosyltransferase (UPRT) exogenous to the cell and operably linked to a promoter that is active or can be activated in said cell.

8. The method according to Claim 7, wherein sequences encoding said UPRT are operably linked to a promoter that can be activated in said cell.

9. The method according to Claim 8, wherein said UPRT is *Toxoplasma gondii* UPRT or a functional derivative thereof.

10. The method according to Claim 7, wherein said small molecule binding partner is biotin.

11. The method according to Claim 10, further comprising the step of binding an avidin to said biotin.

12. The method according to Claim 11, wherein said avidin is conjugated to an insoluble substrate for affinity chromatography, and wherein said biosynthetically labelled RNA is separated from non-labelled RNA.

13. The method according to Claim 12, wherein said separated RNA is amplified.

14. The method according to Claim 11, wherein said avidin is conjugated to a detectable label.

15. The method according to Claim 14, wherein said detectable label is a fluorochrome, radiolabel, heavy metal label or enzyme conjugate.

## Patentansprüche

1. Verfahren zur biosynthetischen Markierung von RNA in einer Zelle von Interesse, wobei das Verfahren Folgendes umfasst:
das Kontaktieren der Zelle in vitro mit einem Uracil-Analogon, das normalerweise in RNA nicht vorliegt und aus 2-Thiouracil, 4-Thiouracil, 2,4-Dithiouracil, 2-Thio-4-desoxyuracil, 5-Ethoxycarbonyl-2-thiouracil, 5-Carboxy-2-thiouracil, 5-(n-Propyl)-2-thiouracil, 6-Methyl-2-thiouracil und 6-(n-Propyl)-2-thiouracil ausgewählt ist, worin die Zelle eine Phosphoribosyltransferase oder eine Nucleosidkinase umfasst, die das Uracil-Analogon spezifisch in das entsprechende Nucleotid inkorporieren kann, worin die Phosphoribosyltransferase oder die Nucleosidkinase exogen zu der Zelle von Interesse ist und worin das Uracil-Analogon in RNA inkorporiert wird, die von der Zelle synthetisiert wird; sowie das Erhalten von RNA, umfassend die Thiol-Gruppierung aus der Zelle.

2. Verfahren nach Anspruch 1, worin das Uracil-Analogon aus 2-Thiouracil, 4-Thiouracil, 2,4-Dithiouracil und Gemischen davon ausgewählt ist.

3. Verfahren nach Anspruch 1, worin das Verfahren weiters das Konjugieren eines Kleinmolekül-Bindungspartners an die Thiolkomponente des Uracil-Analogons umfasst.

4. Verfahren nach Anspruch 3, worin der Kleinmolekül-Bindungspartner Biotin ist und/oder worin der Kleinmolekül-Bindungspartner an eine detektierbare Markierung konjugiert ist, die z.B. ein Fluoreszenzfarbstoff, eine Radiomarkierung, eine Schwermetallmarkierung oder ein Enzymkonjugat ist.

5. Verfahren nach Anspruch 4, weiters umfassend den Schritt der Bindung eines spezifischen Bindungspartners an den Kleinmolekül-Bindungspartner, gegebenenfalls worin entweder:
der spezifische Bindungspartner an ein unlösliches Substrat für eine Affinitätschromatographie konjugiert ist und worin die biosynthetisch markierte RNA von nicht-markierter RNA getrennt wird; oder
der spezifische Bindungspartner an eine detektierbare Markierung konjugiert ist, z.B. einen Fluoreszenzfarbstoff, eine Radiomarkierung, eine Schwermetallmarkierung oder ein Enzymkonjugat.

6. Verfahren nach Anspruch 5, worin die getrennte RNA umkehrtranskribiert wird oder worin die getrennte RNA amplifiziert wird; und/oder worin die getrennte RNA, gegebenenfalls während der Amplifikation, mit einer detektierbaren Markierung durch z.B. Endmarkierung oder durch Umkehrtranskriptase markiert wird.

7. Verfahren nach Anspruch 1, worin die Phosphoribosyltransferase eine Uracil-Phosphoribosyltransferase (UPRT) ist, die exogen zu der Zelle ist und operabel an einen Promotor gebunden ist, der aktiv ist oder der in der Zelle aktiviert werden kann.

8. Verfahren nach Anspruch 7, worin Sequenzen, die für die UPRT kodieren, operabel an einen Promotor gebunden sind, der in der Zelle aktiviert werden kann.

9. Verfahren nach Anspruch 8, worin die UPRT Toxoplasma-gondii-UPRT oder ein funktionelles Derivat davon ist.

10. Verfahren nach Anspruch 7, worin der Kleinmolekül-Bindungspartner Biotin ist.

11. Verfahren nach Anspruch 10, weiters umfassend den Schritt der Bindung eines Avidins an das Biotin.

12. Verfahren nach Anspruch 11, worin das Avidin an ein unlösliches Substrat für eine Affinitätschromatographie konjugiert ist und worin die biosynthetisch markierte RNA von nicht-markierter RNA getrennt wird.

13. Verfahren nach Anspruch 12, worin die getrennte RNA amplifiziert wird.

14. Verfahren nach Anspruch 11, worin das Avidin an eine detektierbare Markierung konjugiert ist.

15. Verfahren nach Anspruch 14, worin die detektierbare Markierung ein Fluoreszenzfarbstoff, eine Radiomarkierung, eine Schwermetallmarkierung oder ein Enzymkonjugat ist.

## Revendications

1. Procédé d'étiquetage biosynthétique du ARN dans une cellule d'intérêt, le procédé comprenant: mettre ladite cellule en contact *in vitro* avec un analogue d'uracil qui n'est normalement pas présent dans l'ARN et est sélectionné parmi 2-thiouracil, 4-thiouracil, 2,4-dithiouracil, 2-thio-4-désoxyuracil, 5-carbéthoxy-2-thiouracil, 5-carboxy-2-thiouracil, 5-(*n*-propyl)-2-thiouracil, 6-méthyl-2-thiouracil et 6-(*n*-propyl)-2-thiouracail, où ladite cellule comprend une phosphoribosyltransférase ou nucléoside kinase apte à incorporer spécifiquement ledit analogue d'uracil dans le nucléotide correspondant, où ladite phosphoribosyltransférase ou nucléoside kinase est exogène à la cellule d'intérêt, et où ledit analogue d'uracil est incorporé dans l'ARN synthétisé par ladite cellule; et obtenir l'ARN comprenant ledit fragment thiol de ladite cellule.

2. Méthode selon la revendication 1, dans laquelle ledit analogue d'uracil est sélectionné parmi 2-thiouracil, 4-thiouracil, 2,4-dithiouracil et leurs mélanges.

3. Méthode selon la revendication 1, dans laquelle ladite méthode comprend en outre la conjugaison d'un partenaire de liaison de petites molécules au composant thiol de l'analogue d'uracil.

4. Méthode selon la revendication 3, dans laquelle ledit partenaire de liaison de petites molécules est la biotine et/ou dans laquelle ledit partenaire de liaison de petites molécules est conjugué à un marqueur détectable qui est, par exemple, un fluorochrome, radiomarqueur, marqueur en métal lourd ou conjugué d'enzyme.

5. Méthode selon la revendication 4, comprenant en outre l'étape consistant à lier un partenaire de liaison spécifique audit partenaire de liaison de petites molécules, optionnellement, dans lequel soit:
ledit partenaire de liaison spécifique est conjugué à un substrat insoluble pour une chromatographie d'affinité, et dans laquelle ledit ARN à étiquetage biosynthétique est séparé de l'ARN non étiqueté; ou
ledit partenaire de liaison spécifique est conjugué à un marqueur détectable, par exemple, un fluorochrome, radiomarqueur, marqueur en métal lourd ou conjugué d'enzyme.

6. Méthode selon la revendication 5, dans laquelle ledit ARN séparé est transcrit inversement ou dans laquelle ledit ARN séparé est amplifié; et/ou dans laquelle ledit ARN séparé est marqué, optionnellement durant l'amplification, avec un marqueur détectable, par exemple par marquage final ou par transcriptase inverse.

7. Méthode selon la revendication 1, dans laquelle ladite phosphoribosyltransférase est une phosphoribosyltransférase d'uracil (UPRT) exogène à la cellule et fonctionnellement liée à un promoteur qui est actif ou peut être activé dans ladite cellule.

8. Méthode selon la revendication 7, dans laquelle des séquences codant pour ladite UPRT sont fonctionnellement liées à un promoteur qui peut être activé dans ladite cellule.

9. Méthode selon la revendication 8, dans laquelle ladite UPRT est *Toxoplasma gondii* UPRT ou un dérivé fonctionnel de celle-ci.

10. Méthode selon la revendication 7, dans laquelle ledit partenaire de liaison de petites molécules est la biotine.

11. Méthode selon la revendication 10, comprenant en outre l'étape de liaison d'une avidine à ladite biotine.

12. Méthode selon la revendication 11, dans laquelle ladite avidine est conjuguée à un substrat insoluble pour la chromatographie d'affinité, et dans laquelle ledit ARN à étiquetage biosynthétique est séparé de l'ARN non étiqueté.

13. Méthode selon la revendication 12, dans laquelle ledit ARN séparé est amplifié.

14. Méthode selon la revendication 11, dans laquelle ladite avidine est conjuguée à un marqueur détectable.

15. Méthode selon la revendication 14, dans laquelle ledit marqueur détectable est un fluorochrome, radiomarqueur, marqueur en métal lourd ou conjugué d'enzyme.
